# EUROPÄISCHE PATENTSCHRIFT

(11) **EP 3 827 775 B1**
(45) Veröffentlichungstag und Bekanntmachung des Hinweises auf die Patenterteilung: **23.02.2022**
(21) Anmeldenummer: 20208106.3
(22) Anmeldetag: 17.11.2020
(51) Int. Cl.: A61B 1/00, A61B 18/14, A61B 1/307, A61B 1/12, A61B 18/00

(54) **TRANSPORTEUR MIT VERRIEGELUNGSEINRICHTUNG**
TRANSPORTER WITH LOCKING DEVICE
TRANSPORTEUR POURVU DE DISPOSITIF DE VERROUILLAGE

(30) Priorität: 29.11.2019 DE 102019132536
(43) Veröffentlichungstag der Anmeldung: 02.06.2021
(73) Patentinhaber: Olympus Winter & Ibe GmbH, 22045 Hamburg (DE)
(72) Erfinder: IPSEN, Hendrik, 22885 Barsbüttel (DE); BROCKMANN, Christian, 21279 Hollenstedt (DE); MIERSCH, Hannes, 22149 Hamburg (DE)
(74) Vertreter: Hoener, Matthias

(56) Entgegenhaltungen:
- DE-A1-102011 011 216
- DE-A1-102015 010 877
- US-A1- 2008 224 427

## Beschreibung

Die Erfindung betrifft einen Transporteur der im Oberbegriff des Anspruches 1 genannten Art.

Transporteure der gattungsgemäßen Art werden in Resektoskopen vor allem in der Urologie bei chirurgischen Arbeiten in der Blase und der Urethra verwendet. Sie werden beispielsweise in Verbindung mit elektrochirurgischen Durchgangsinstrumenten zur Resektion und Vaporisation von Gewebe, zum Beispiel von Gewebe im unteren Harntrakt verwendet. Dazu umfassen die Resektoskope ein längsverschiebbares Elektrodeninstrument, das nach dem Einführen des Resektoskops mit seinem distalen Arbeitsende aus dem distalen Ende des Schaftrohres des Resektoskops hervorgeschoben werden kann.

Das Elektrodeninstrument ist an seinem proximalen Ende mit dem Transporteur des Resektoskops verbunden, von dem es in Längsrichtung verschoben werden kann, um die Schneidbewegung auszuführen. Der Transporteur ist üblicherweise abnehmbar am proximalen Ende des Schaftrohres des Resektoskops befestigt.

Der Transporteur weist einen längsverschiebbaren Schlitten auf, mit dem das Elektrodeninstrument zur gemeinsamen Längsbewegung koppelbar ist. Die Betätigung des Transporteurs erfolgt üblicherweise durch die Finger einer Hand, die einerseits am Schlitten und andererseits an den feststehenden Teilen des Transporteurs angreifen, die mit dem Führungsrohr fest verbunden sind. Dabei kann die Bewegung des Transporteurs beispielsweise gegen eine rückstellende Feder erfolgen, die als Schenkelfeder oder Blattfeder ausgebildet sein kann. Beispielhafte Instrumente sind in der DE 10 2011 011 216 A1, US 2004/0242959 A1, US 6358200 und DE 35 00 527 A1 beschrieben.

Die Kopplung zwischen dem Transporteur und von ihm längsverschiebbaren Arbeitsinstrumenten, wie einem Elektrodeninstrument, oder anderen stationären Systemkomponenten, wie dem Schaft, erfolgt üblicherweise über Rastverbindungen. Wird beispielsweise das proximale Ende des Elektrodeninstruments mit dem entsprechenden Verbindungspartner in dem Transporteur in Kontakt gebracht, so erfolgt durch das Einführen des Elektrodeninstruments in der Regel eine automatische Einrastung des Instruments. Die Entrastung des Elektrodeninstruments muss dagegen üblicherweise manuell unterstützt werden.

Die Verrastung des Elektrodeninstruments erfolgt derzeit durch einen Hinterschnitt eines länglichen Rastelements in einer dafür vorgesehenen Nut in dem Instrumentenschaft. Das Rastelement ist hierfür quer zur Längsrichtung des Arbeitsinstruments und des Resektoskopschaftes in dem Transporteur angeordnet. Durch eine Einführschräge am Rastelement erzeugt der Schaft des eingeführten Arbeitsinstruments eine Kraft, die das Rastelement entlang seiner eigenen Längsachse und quer zur Längsachse des Arbeitsinstruments verschiebt. Durch einen mittels eines Federelements ausgeübten Gegendruck wird das Rastelement anschließend in seine Ausgangsstellung zurückgedrückt und der Hinterschnitt in der Rastverbindung sichergestellt. Das in der Rastverbindung verwendete Federelement kann z. B. eine Druckfeder oder ein Elastomer-Formteil sein.

Um auch ohne eingerastetes Arbeitsinstrument eine definierte Ruhelage des Rastelements zu gewährleisten und eine unerwünschte Demontage zu verhindern, umfasst die Rastverbindung üblicherweise ein fest verbautes Sperrelement, das in einer Durchbrechung des Rastelements verbaut ist und ein unerwünschtes Herausfallen des Rastsystems aus dem Transporteur verhindert.

Die Entrastung des Arbeitsinstruments oder der anderen verrasteten Systemkomponenten erfolgt durch manuelle, d.h. händisch durch das Bedienpersonal durchgeführte, Verschiebung des Rastelements quer zur Längsachse des Transporteurs.

Da das Rastelement bewegbar in einem Kanal innerhalb eines Verbindungskörpers des Transporteurs angeordnet ist, besteht rund um das Rastelement ein enger Spalt, der während der Reinigung und der Sterilisation nur schwer zu erreichen ist. Die Verschiebungen des Rastelements können ferner zu Verschmierungen von Verunreinigungen in diesem Spalt führen. Darüber hinaus ist der Kanal, in dem das Rastelement innerhalb des Verbindungskörpers angeordnet ist, häufig auf beiden Seiten des Transporteurs geschlossen. Auf der einen Seite wird der Kanal dabei durch eine Seitenwand des Transporteurs geschlossen, auf der anderen Seite durch das Federelement, zum Beispiel in Form eines Elastomer-Formteils, oder ein Betätigungselement. Dies erschwert die Reinigung zusätzlich und schafft weitere Hohlräume, die schwer zu reinigen sind. Auch in der für das Sperrelement vorgesehenen Durchbrechung des Rastelements können sich Verunreinigungen ansammeln. Die Verwendung eines Elastomer-Formteils als Federelement birgt zudem das Risiko, dass durch die entstehende Verformung bei der Betätigung des Rastelements Verunreinigungen in dem Federelement an Stellen eingeschlossen werden können, die schlecht oder gar nicht zu reinigen sind.

Aufgabe der vorliegenden Erfindung ist es daher, einen Transporteur bereitzustellen, der leichter und zuverlässiger zu reinigen ist, insbesondere im Bereich des für die Verrastung von Arbeitsinstrumenten und anderen Systemkomponenten vorgesehenen Rastsystems.

### Beschreibung

Gelöst wird diese Aufgabe durch einen Transporteur mit den Merkmalen von Anspruch 1 sowie durch ein Resektoskop mit den Merkmalen von Anspruch 14.

Die Erfindung betrifft insbesondere in einem ersten Aspekt einen Transporteur für ein Resektoskop für die endoskopische Chirurgie, wobei der Transporteur einen Verbindungskörper und zur Verbindung mit einer Systemkomponente in dem Verbindungskörper ein Rastsystem aufweist, das ein langgestrecktes, vorzugsweise quer zur Längsachse des Transporteurs angeordnetes Rastelement mit einer Eingriffsöffnung oder einer Eingriffsnut und ein Federelement umfasst, dadurch gekennzeichnet, dass zwischen Rastelement und Verbindungskörper ein oder mehrere, parallel zur Längsachse des Rastelements angeordnete Spülkanäle ausgebildet sind.

In einer bevorzugten Ausführungsform betrifft die Erfindung einen Transporteur für ein Resektoskop für die endoskopische Chirurgie, wobei der Transporteur zur Steuerung der Längsverschiebung eines Arbeitsinstruments, das einen langgestreckten Schaftabschnitt aufweist, ausgebildet ist und zur Verbindung mit dem proximalen Endbereich des Arbeitsinstruments ein Rastsystem aufweist, das ein langgestrecktes, quer zur Längsachse des Transporteurs angeordnetes Rastelement mit einer seitlichen Eingriffsöffnung und ein Federelement umfasst, dadurch gekennzeichnet, dass das Rastelement ein oder mehrere, parallel zur Längsachse des Rastelements angeordnete Spülnuten aufweist.

In einem verwandten Aspekt betrifft die Erfindung ein Resektoskop zur Verwendung in der endoskopischen Chirurgie, dadurch gekennzeichnet, dass es einen erfindungsgemäßen Transporteur und vorzugsweise eine in dem Transporteur verrastete Systemkomponente umfasst, besonders bevorzugt ein Arbeitsinstrument.

Durch die erfindungsgemäße Einlassung von Spülnuten in das Rastelement und/oder den Verbindungskörper werden in dem Transporteur von außen zugängliche Spülkanäle gebildet, welche die Reinigung und Sterilisation des Rastsystems erleichtern und verbessern. Alle Elemente des Rastsystems können während der Reinigung von Spülflüssigkeit umspült bzw. bei der Sterilisation von Sterilisationsgas erreicht werden. Die Gefahr einer andauernden Kontamination der Instrumente ist damit gegenüber dem Stand der Technik deutlich gesenkt.

Das erfindungsgemäß modifizierte Rastsystem ist für Transporteure geeignet, die in der endoskopischen Chirurgie verwendet werden. Für diese Instrumente ist ein hoher Reinheitsgrad und Sterilität besonders wichtig. Die Transporteure sind in der Regel Teile eines Resektoskops, das darüber hinaus in üblicher Ausbildungsweise noch ein Schaftteil mit einem rohrartigen Schaft und eine durch den Transporteur in den Schaftteil einführbare langgestreckte Optik aufweist. Die Teile des Resektoskops können den Erfordernissen des jeweiligen Eingriffs angepasst werden und daher variieren.

Neben Transporteur, Schaftteil und Optik können die Resektoskope weitere Teile aufweisen, beispielsweise ein Leuchtmittel, wie ein Lichtleitfaserbündel, und unterschiedliche Arbeitsinstrumente (Durchgangsinstrumente), die durch den Schaft des Resektoskops zur Eingriffsstelle geführt werden und dort beispielsweise zur Manipulation von Gewebe verwendet werden können.

Der Transporteur weist einen Verbindungskörper auf, in dem das erfindungsgemäße Rastsystem angeordnet ist. Der Verbindungskörper grenzt somit unmittelbar an das Rastsystem an, insbesondere an das Rastelement des Rastsystem aber auch an die übrigen Komponenten des Rastsystems. Soll der Transporteur zur Längsverschiebung der verrasteten Systemkomponente ausgebildet sein, so kann der Verbindungskörper beispielsweise der Schlitten des Transporteurs sein, oder ein Teil des Schlittens.

Der Transporteur ist mittels des Rastsystems zur Verbindung mit einer Systemkomponente ausgebildet. Vorzugsweise ist insbesondere der Verbindungskörper des Transporteurs zur Verbindung mit einer Systemkomponente ausgebildet. Dies bedeutet, dass die Systemkomponente mittels des Rastsystems in dem Transporteur, insbesondere dessen Verbindungskörper, lösbar gehalten werden kann.

Das Rastsystem ist zur Verrastung unterschiedlicher Teile geeignet. Die Systemkomponente kann somit jegliche Komponente (Teil) eines Resektoskops sein, die lösbar mit dem Transporteur, insbesondere dessen Verbindungskörper, verbunden werden soll. Dabei ist die Systemkomponente kein Teil des Transporteurs. Die Systemkomponente kann beispielsweise ausgewählt sein aus der Gruppe bestehend aus Arbeitsinstrument (Durchgangsinstrument), Optik, Schaftrohrsystem (z.B. Hüllrohr und/oder Innenrohr) und anderen. In einer bevorzugten Ausführungsform ist die Systemkomponente ein Arbeitsinstrument. In einer anderen Ausführungsform ist die Systemkomponente eine Optik.

Es versteht sich entsprechend, dass der erfindungsgemäße Transporteur nicht nur ein hierin beschriebenes Rastsystem umfassen kann, sondern auch mehrere, wobei die verschiedenen Rastsysteme jeweils zur Verrastung unterschiedlicher Systemkomponenten ausgebildet und angeordnet sind. Der Transporteur kann beispielsweise ein, zwei, drei oder mehr der hierin beschriebenen Rastsysteme umfasst, vorzugsweise zwei oder mehr.

Wie erwähnt ist die verrastbare Systemkomponente vorzugsweise ein Arbeitsinstrument. Diese sind in der Regel längsverschiebbar in einem Resektoskop angeordnet, um dem medizinischen Fachpersonal einen flexiblen Eingriff an der Eingriffsstelle zu ermöglichen. Der Transporteur kann entsprechend zur Steuerung der Längsverschiebung eines Arbeitsinstruments ausgebildet sein. Geeignete Arbeitsinstrumente, die für einen Eingriff in der endoskopischen Chirurgie verwendet werden können, sind Fachleuten bekannt. Das Arbeitsinstrument kann beispielsweise ein Elektrodeninstrument oder ein Steinfanginstrument sein, wobei das hierin beschriebene Arbeitsinstrument bevorzugt ein Elektrodeninstrument ist.

Das Elektrodeninstrument weist einen langgestreckten Schaftabschnitt (Schaftteil) auf und ist als Durchgangsinstrument für ein Resektoskop ausgebildet, d.h. als Instrument, das durch ein resektoskopisches Schaftrohr in eine Körperöffnung einführbar ist. Das Elektrodeninstrument weist an seinem distalen Ende eine mit Hochfrequenzstrom beaufschlagbare Elektrode auf. Bei der Elektrode kann es sich um eine Schneidschlinge, einen Vaporisationsknopf (PlasmaButton) oder andere handelsübliche Elektroden handeln. Bevorzugt ist die Elektrode eine Schneidschlingenelektrode. Entsprechende Elektroden und Elektrodeninstrumente sind Fachleuten bekannt.

Das Elektrodeninstrument kann ein bipolares Elektrodeninstrument sein, das die Elektrode als Teil einer Elektrodenanordnung umfasst. In diesem Falle wird das Elektrodeninstrument zum Beispiel eine zweite Elektrode im distalen Endbereich des Elektrodeninstruments umfassen, die als Neutralelektrode ausgebildet ist. Alternativ kann die zweite Elektrode (Neutralelektrode) auch an anderen Elementen des distalen Endbereichs des Resektoskops angeordnet sein. Selbstverständlich kann das Elektrodeninstrument auch als monopolares Instrument ausgebildet sein.

Die Systemkomponente kann einen langgstreckten Schaftabschnitt aufweisen. Vorzugsweise ist die Längsachse der Systemkomponente parallel zur Längsachse des Resektoskopsschaftes bzw. des Transporteurs. Der proximale Endbereich der Systemkomponente kann mit dem Rastsystem verbunden sein. Es ist aber auch denkbar, eine laterale Seite der Systemkoponente mit einer am bzw. im Verbindungskörper seitlich angeordneten Eingriffsöffnung bzw. Eingriffsnut zu verbinden, d.h. zu verrasten. Bevorzugt ist aber das Rastsystem zur Verbindung mit dem proximalen Endbereich der Systemkomponente ausgebildet. In dieser Ausführungsform ist der proximale Endbereich der Systemkomponente in die Eingriffsöffnung bzw. Eingriffsnut des Rastsystems einführbar.

Ist die Systemkomponente ein Arbeitsinstrument, so kann das Arbeitsinstrument innerhalb des Schaftes des Resektoskops durch den Transporteur längsverschiebbar sein, d. h. in axialer Richtung nach distal und proximal beweglich. Zum Anschluss an das Resektoskop weist das Arbeitsinstrument den langgestreckten Schaftabschnitt auf, der zur Herstellung einer bewegungsgekoppelten Verbindung an seinem proximalen Endbereich an einem von dem Transporteur umfassten Schlitten verrastbar ist. Der Schlitten gleitet typischerweise auf einem Rohr und wird über eine Federeinheit federvorgespannt in einer Ruhestellung gehalten. So kann die Arbeitseinheit, z. B. die Elektrode, am distalen Ende auf zu schneidendes Gewebe zu- oder von diesem wegbewegt werden, ohne das gesamte Resektoskop bewegen zu müssen. Darüber hinaus ist es durch die Längsverschiebbarkeit des Arbeitsinstruments beispielsweise möglich, zwischen einer distalen Elektrode und dem Isoliereinsatz Gewebe einzuklemmen und von der Eingriffsstelle zu entfernen.

Der proximale Endbereich des Schaftabschnitts des Arbeitsinstruments ist mittels eines Rastsystems mit dem Transporteur verbunden. Das Rastsystem ist Teil des Transporteurs und vorzugsweise innerhalb des Schlittens des Transporteurs angeordnet. Auf diese Weise kann das Arbeitsinstrument nach dem Verrasten des Instruments innerhalb des Schlittens mit diesem zusammen bewegt werden.

Das Rastsystem der erfindungsgemäßen Transporteure umfasst stets ein Rastelement und ein Federelement und kann darüber hinaus optional ein Sperrelement umfassen. Das Rastsystem ist innerhalb des und an dem Transporteur so angeordnet, dass das Rastelement und optional das Sperrelement zum größten Teil, d.h. zu etwa 50% oder mehr innerhalb des Transporteurs angeordnet sind. Dazu weist der Transporteur eine entsprechende kanalförmige Aushöhlung (Kanal) auf. Diese ist mindestens auf einer Seite des Transporteurs offen, vorzugsweise auf beiden Seiten. Die Aushöhlung ist in etwa form- und größenkomplementär zu dem Rastsystem. Wie oben beschrieben, bestehen allerdings in den Randbereichen des Rastelements, des Federelements und des Sperrelements durchaus freie Spalte, die im Rahmen der Fertigung entstehen und/oder zur Gewährleistung einer freien Dreh- und Verschiebbarkeit oder anderer Funktionen des Rastsystems notwendig sind.

Das Rastelement ist langgestreckt und quer zur Längsachse des Transporteurs angeordnet. Das Rastelement kann einen Schaftteil aufweisen und an einem Ende einen Kopfteil, der einen größeren Durchmesser aufweist als der Schaftteil. Dieser Kopfteil kann beispielsweise zur Abstützung des Federelements dienen. Schaft- und Kopfteil weisen vorzugsweise einen im Wesentlichen runden Querschnitt, ggfs. mit Ausnehmungen für die Spülnuten, auf. Das Rastelement kann somit, mit anderen Worten, in seinem Schaftteil eine im Wesentlichen zylindrische Form, ggfs. mit Ausnehmungen für die Spülnuten und die Sperrnut aufweisen.

Das Rastelement weist in seiner Mantelfläche, d.h. im Verhältnis zu seiner Längsachse seitlich (lateral), eine oder mehrere seitliche Eingriffsöffnungen und/oder eine oder mehrere seitliche Eingriffsnuten auf. Eine der Eingriffsöffnungen oder eine der Eingriffsnuten ist für die Einführung des eines Teils der Systemkomponente, vorzugsweise des proximalen Endbereichs des Arbeitsinstruments, angeordnet und geeignet.

Die Eingriffsöffnung kann kanalartig ausgebildet sein und einen im Wesentlichen zylindrischen Innenraum aufweisen. Die Eingriffsöffnung kann den Schaftteil des Rastelements kanalartig durchspannen. Die Eingriffsöffnung kann somit an beiden Seiten des Rastelements eine Öffnung erzeugen. Alternativ kann die Eingriffsöffnung an der dem Arbeitsinstrument gegenüberliegenden Seite geschlossen sein.

Die Eingriffsnut kann einen U- oder teilkreisförmigen Querschnitt aufweisen und den Schaftteil des Rastelements durchspannen. Die Nut ist vorzugsweise an beiden Enden entlang ihrer Längsrichtung offen, d.h. von einem Gas oder einer Flüssigkeit durchströmbar.

Die Eingriffsöffnung bzw. die Eingriffsnut sind quer zur Längsachse des Rastelements angeordnet.

Das Rastelement kann weitere Eingriffsöffnungen aufweisen, beispielsweise für den Eingriff eines Sperrelements. Erfindungsgemäß ist es jedoch bevorzugt, dass ein Sperrelement in einer Sperrnut in der Außenwand des Rastelements angeordnet ist, wie an anderer Stelle hierin beschrieben.

Um das Einführen und automatische Einrasten der Systemkomponente, vorzugsweise des Arbeitsinstruments, in das Rastelement zu ermöglichen, kann die Eingriffsöffnung bzw. die Eingriffsnut einen teilweise trichterförmigen Eingangsbereich aufweisen, d.h. einen einseitig schrägen Eingangsbereich. In diesem Eingangsbereich ist die Eingriffsöffnung bzw. die Eingriffsnut verbreitert ausgebildet. An einer Schräge des Eingangsbereichs kann das proximale Ende des Arbeitsinstruments während des Einführens entlang gleiten und dabei das Rastelement derart seitlich, d.h. quer zur Längsachse des Transporteurs verschieben, dass das als Druckfeder ausgebildete Federelement komprimiert wird.

Die für die Systemkomponente, vorzugsweise den proximalen Endbereich des Arbeitsinstruments, vorgesehene Eingriffsöffnung oder Eingriffsnut kann darüber hinaus ein oder mehrere Rastelemente oder Nuten umfassen, die mit einem Rastelement oder einer Nut am Arbeitsinstrument eine Rastverbindung eingehen. Auf diese Weise kann ein geeigneter Hinterschnitt in der Rastverbindung sichergestellt werden.

Zwischen Rastelement und Verbindungskörper sind erfindungsgemäß ein oder mehrere, parallel zur Längsachse des Rastelements angeordnete Spülkanäle ausgebildet. Die Spülkanäle können entweder durch die Ausbildung von Spülnuten in der Außenwand des Rastelements oder durch Ausbildung von Spülnuten in der an das Rastelement angrenzenden Innenfläche des Verbindungskörpers ausgebildet werden. Auch eine Kombination von Spülnuten an den beiden Teilen ist denkbar und im Rahmen der Erfindung möglich. Somit weist in einer Ausführungsform das Rastelement ein oder mehrere, parallel zur Längsachse des Rastelements angeordnete Spülnuten auf. Alternativ oder zusätzlich kann der Verbindungskörper ein oder mehrere, parallel zur Längsachse des Rastelements und, vorzugsweise, angrenzend an das Rastelement angeordnete Spülnuten aufweisen. Es ist im Rahmen der Erfindung auch denkbar, in beiden Teilen Spülnuten derart auszubilden, dass jeweils eine Spülnut des Rastelements an eine Spülnut des Verbindungskörpers angrenzt.

Die Spülnuten können jeweils einen teilkreisförmigen, z. B. einen halbkreisförmigen, oder einen U-förmigen Querschnitt aufweisen. Die Spülnuten sind dazu geeignet, einen Flüssigkeitskanal zu erzeugen, über den zwischen dem Rastelement und den übrigen Teilen des Transporteurs entstehende Spalten mit Spülflüssigkeit ausgespült werden können. Dazu sind die Spülnuten zur Ein- bzw. Ausleitung von Spülflüssigkeit vorzugsweise an beiden Enden des langgestreckten Rastelements bzw. des angrenzenden Verbindungskörpers offen. Die beiden Enden des Rastelements sind die entlang der Längsachse des Rastelements liegenden Enden. Innerhalb des Transporteurs sind diese Enden zu den Seiten des Transporteurs hin orientiert (lateral). Durch die Spülnuten ist entsprechend Spülflüssigkeit und/oder Gas (Sterilisationsgas) von einer lateralen Seite des Transporteurs auf die kontralaterale Seite des Transporteurs leitbar.

Das Rastelement bzw. der Verbindungskörper kann eine oder mehrere Spülnuten aufweisen, d.h. beispielsweise eine, zwei, drei, vier oder mehr Spülnuten. In einer bevorzugten Ausführungsform weist das Rastelement bzw. der Verbindungskörper, vorzugsweise das Rastelement, vier Spülnuten auf. Die Spülnuten können um die Längsachse des Rastelements herum auf seinem Umfang bzw. auf dem Innenumfang der Aushöhlung des Verbindungskörpers verteilt sein. Dabei sind alle Spülnuten bevorzugt äquidistant voneinander beabstandet. So können in einem Rastelement bzw. Verbindungskörper mit vier Spülnuten die Nuten beispielsweise bei 0°, 90°, 180° und 270° auf dessen Umfang bzw. Innenumfang der Aushöhlung angeordnet sein. Bevorzugt sind jeweils einander gegenüberliegende Paare von Spülnuten auf dem Rastelement bz.w dem Innenumfang der Aushöhlung angeordnet.

Das Rastsystem kann ferner ein langgestrecktes Sperrelement umfassen, das quer zur Längsachse des Rastelements in einer Sperrnut in der Außenwand des Rastelements angeordnet ist. Das Sperrelement verhindert in Abwesenheit des Arbeitsinstruments ein Herausfallen des Rastsystems aus dem Transporteur. Das Sperreleement weist einen langgestreckten Schaft auf, dessen eines Ende - vorzugsweise das proximale Ende - zumindest teilweise in einer Öffnung des Transporteurs bzw. des Schlittens angeordnet ist, die nicht Teil des Rastelements ist. Dieses Ende ist mit anderen Worten in einem das Rastsystem umschließenden Körper des Transporteurs angeordnet. Das andere Ende des Sperrelements - vorzugsweise das distale Ende - ist in einer Sperrnut des Rastelements angeordnet. Ein Teil des Sperrelements liegt somit in Abwesenheit des Arbeitsinstruments an einer Seitenfläche der Sperrnut an und verhindert so eine weitere Bewegung des Rastelements in Richtung der von dem Federelement ausgeübten Federkraft. Das Sperrelement kann ein von außerhalb des Verbindungskörpers zugängliches Kopfteil mit einem breiteren Durchmesser umfassen.

Die Sperrnut ist seitlich in dem Schaftteil des Rasteleements angeordnet. Dabei ist die Sperrnut vorzugsweise in dem Endbereich des Rastelements lokalisiert, der nicht an das Federelement angrenzt. Die Sperrnut kann einen teilkreisförmigen Querschnitt aufweisen. Sie grenzt bevorzugt an eine oder mehrere Spülnuten an und bildet mit der oder den Spülnuten einen Spülkanal. Diese durch die Spülnuten und, optional, die Sperrnut erzeugten Spülkanäle erstrecken sich vorzugsweise über die gesamte Länge des Rastelements.

Die Sperrnut ist mindestens abschnittsweise form- und größenkomplementär zu dem Sperrelement ausgebildet und angeordnet. Dabei weist die Sperrnut jedoch in Längsrichtung des Rastelements einen größeren Durchmesser auf, als das Sperrelement breit ist, um eine gewünschte Verschiebbarkeit des Rastelements in Längsrichtung des Rastelements zu gewährleisten.

Das Rastsystem umfasst ferner ein Federelement. Das Federelement übt eine Federkraft in Richtung einer Außenseite, beispielsweise der einem Sperrelement abgewandten Außenseite, des Transporteurs aus. Wenn der Schaft des Arbeitsinstruments in die Eingriffsöffnung eingeführt ist, wird hierdurch zum Sicherstellen des Hinterschnitts ein Druck auf den Schaft ausgeübt (Klemmwirkung). Das Federelement ist vorzugsweise eine Druckfeder, beispielsweise eine Schraubendruckfeder oder ein elastisches Formteil. In bevorzugten Ausführungsformen ist das Federelement eine Schraubendruckfeder. Da diese keine zusätzlichen Hohlräume in dem Rastsystem erzeugt, ist das so erhaltene Rastsystem leichter und zuverlässiger zu reinigen. Es ist besonders bevorzugt, dass das Federelement, insbesondere die Schraubendruckfeder, aus Metall ist. Geeignete Federelemente sind Fachleuten bekannt.

### Kurze Beschreibung der Figuren

In den Zeichnungen sind Ausführungsbeispiele der Erfindung schematisch dargestellt. Es zeigen:
- **Fig. 1**: eine seitliche, schematische Schnittdarstellung eines erfindungsgemäßen Resektoskops, das einen Transporteur und ein mittels des Schlittens des Transporteurs längsverschiebbares Elektrodeninstrument aufweist;
- **Fig. 2**: eine seitliche, schematische Schnittdarstellung eines erfindungsgemäßen Transporteurs mit einem mittels des Schlittens des Transporteurs längsverschiebbares Elektrodeninstrument;
- **Fig. 3**: eine schematische Schnittdarstellung eines Teils eines Transporteurs aus dem Stand der Technik, der ein Rastsystem mit einem Rastelement, Federelement und einem Sperrelement, wobei das Rastelement keine Spülkanäle aufweist;
- **Fig. 4**: eine um 90° gedrehte schematische Schnittdarstellung des Teils aus Fig. 3;
- **Fig. 5**: eine schematische Schnittdarstellung des Rastsystems eines erfindungsgemäßen Transporteurs aus Längsrichtung des Transporteurs, in der die Spülnuten des Sperrelements erkennbar sind; und
- **Fig. 6**: eine schematische Schnittdarstellung des Rastsystems eines alternativen, erfindungsgemäßen Transporteurs aus Längsrichtung des Transporteurs.

### Ausführungsbeispiele

Weitere Vorteile, Kennzeichen und Merkmale der vorliegenden Erfindung werden bei der nachfolgenden detaillierten Beschreibung von Ausführungsbeispielen anhand der beigefügten Zeichnungen deutlich. Allerdings ist die Erfindung nicht auf diese Ausführungsbeispiele beschränkt.

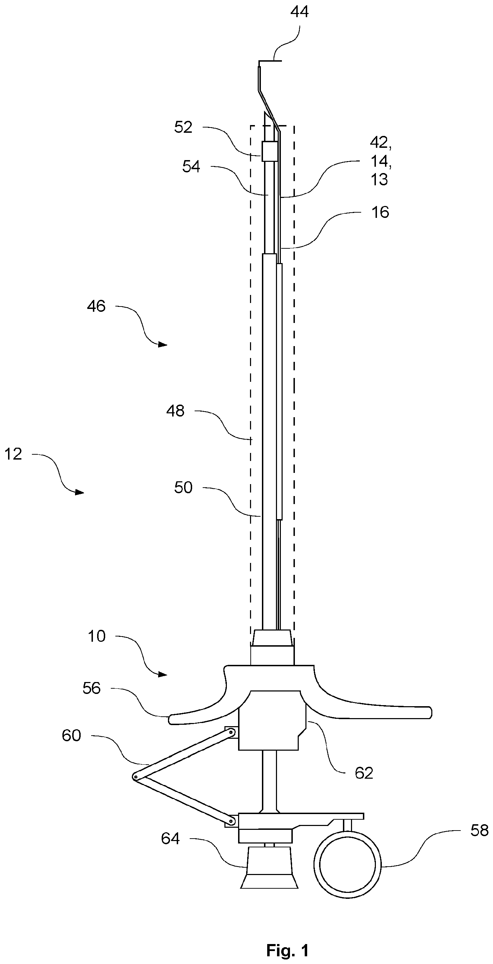

Fig. 1 zeigt eine seitliche, schematische Schnittdarstellung eines erfindungsgemäßen Resektoskops 12, das einen Transporteur 10 und ein mittels des Schlittens 62 des Transporteurs 10 längsverschiebbares Elektrodeninstrument 42 aufweist. Das Elektrodeninstrument 42 ist die Systemkomponente 13 des Transporteurs 10, die mittels eines Rastsystems 18 an dem Transporteur 10 lösbar gehalten werden soll.

Das Resektoskop 12 weist einen Schaft 46 auf, der ein gestrichelt dargestelltes Schaftrohrsystem 48 umfasst, das in nicht dargestellter Weise aus einem Hüllrohr und einem Innenrohr besteht. Innerhalb des Schaftrohrsystems 48 verläuft ein Optikführungsrohr 50 und innerhalb des Optikführungsrohrs 50 eine Optik 54. Zwischen Optikführungsrohr 50 und Schaftrohrsystem 48 verläuft ein Arbeitsinstrument 14, das als Elektrodeninstrument 42 ausbildet ist. Darüber hinaus verläuft im Inneren oder außerhalb des Optikführungsrohres 50 ein nicht dargestelltes Beleuchtungsmittel, zum Beispiel in Form eines Lichtleitfaserbündels. Darüber hinaus können weitere hier nicht dargestellte Elemente in den Resektoskopen 12 verlaufen, wie beispielsweise ein separates Spülrohr und dergleichen. Das Schaftrohrsystem 48 umfasst in seinem distalen Endbereich in hier nicht dargestellter Weise Öffnungen durch die verunreinigte Spülflüssigkeit in den Zwischenraum zwischen Hüllrohr und Innenrohr einströmen und durch den Resektoskopschaft 46 abfließen kann.

Das Elektrodeninstrument 42 ist mittels eines Führungselements 52, das einen teilkreisförmigen Querschnitt aufweist, gegen Verschiebungen abweichend von der Längsrichtung des Schaftes 46, zum Beispiel quer zur Längsrichtung, geschützt. Das Führungselement 52 stützt sich an der Optik 54 ab. Das Elektrodeninstrument 42 ist längsverschiebbar an dem Optikführungsrohr 50 gelagert und weist einen langgestreckten Schaft 16 auf.

Das proximale Ende des Schaftes 16 des Elektrodeninstruments 42 ist in Fig. 1 nicht dargestellter Weise im Inneren des Schlittens 62 des Transporteurs 10 verrastet. Der Schlitten 62 kann somit in der gezeigten Ausführungsform als Verbindungskörper 15 angesehen werden. Das Elektrodeninstrument 42 kann durch Betätigung der Griffteile 56, 58 zwangsgeführt axial in distale und proximale Richtung bewegt werden. Dabei kann es über das distale Ende des Optikführungsrohrs 50 und des Schaftrohrsystems 48 hinausgeschoben werden. So wird es dem Operateur ermöglicht, auch weiter von der Resektoskopspitze entferntes Gewebe zu manipulieren. Das Elektrodeninstrument 42 weist an seinem distalen Ende eine Elektrode 44 auf, die als Schneidschlinge ausgebildet ist und mittels derer Gewebe durch elektrochirurgische Ablation entfernt werden kann. Hierbei wird an die Elektrode 44 eine hochfrequente, elektrische Spannung angelegt, um Gewebe zu zerschneiden.

Das dargestellte Resektoskop 10 weist einen passiven Transporteur 10 auf, bei dem der Schlitten 62 durch Relativbewegung der proximal von dem Resektoskopschaft 46 angeordneten Griffteile 56 und 58 zueinander gegen eine von einer Federbrücke 60 aufgebrachte Federkraft in distale Richtung gegen das distale, erste Griffteil 56 verschoben wird. Bei der Verschiebung des Schlittens 62 in distale Richtung gegen das Griffteil 56 wird das Elektrodeninstrument 42 durch die in Fig. 1 nicht dargestellte Verrastung in dem Rastsystem 18 zwangsgeführt nach distal verschoben. Bei einer Entlastung der Griffteile 58, 56 zwingt die von der Federbrücke 60 erzeugte Federkraft den Schlitten 62 zurück in seine Ruheposition, wobei das Elektrodeninstrument 42 in proximale Richtung gezogen wird. Bei der Rückverschiebung des Schlittens 62 kann ohne Handkraft des Operateurs, also passiv, ein elektrochirurgischer Eingriff mit dem Elektrodeninstrument 42 vorgenommen werden.

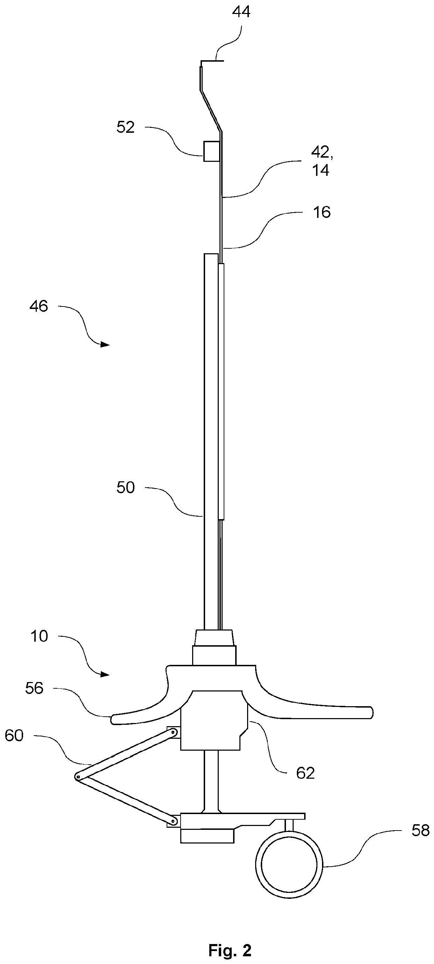

Fig. 2 zeigt eine seitliche, schematische Schnittdarstellung des in Fig. 1 abgebildeten erfindungsgemäßen Transporteurs 10 mit dem mittels des Schlittens 62 des Transporteurs 10 längsverschiebbaren Elektrodeninstrument 42 und dem Optikführungsrohr 50, aber ohne das Schaftrohrsystem 48 des Resektoskops 12 und ohne die Optik 54. Es ist erkennbar, dass das Elektrodeninstrument 42 mit dem proximalen Endbereich seines Schaftabschnitts 16 in den Schlitten 62 des Transporteurs 10 eingeführt ist und von diesem gehalten wird.

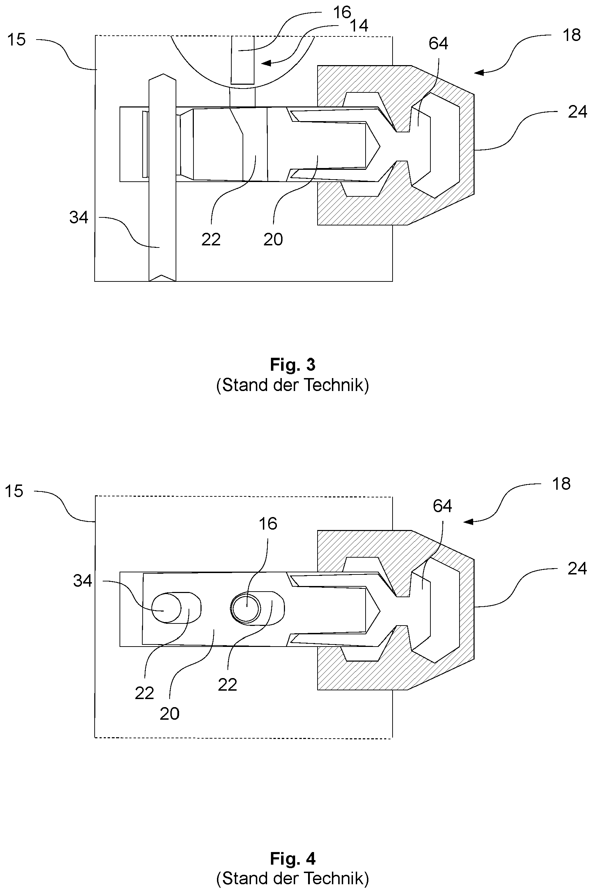

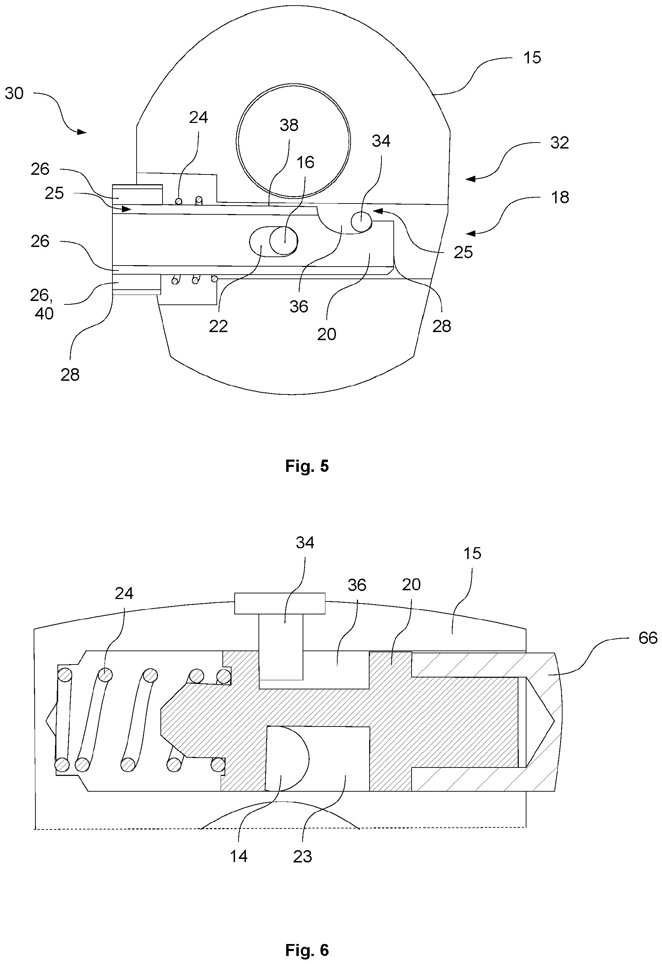

Fig. 3 und 4 zeigen schematische Schnittdarstellungen eines Teils eines Transporteurs 10 aus dem Stand der Technik, der ein Rastsystem 18 mit einem Rastelement 20, Federelement 24 und einem Sperrelement 34 umfasst. Der Transporteur 10 aus dem Stand der Technik unterscheidet sich hauptsächlich dadurch von dem in Fig. 5 abgebildeten erfindungsgemäßen Transporteur 10, dass das Rastelement 20 des in Fig. 3 und 4 gezeigten Transporteurs 10 keine Spülkanäle aufweist. Fig. 4 zeigt eine um 90° gedrehte schematische Schnittdarstellung des Teils aus Fig. 3.

Es ist der proximale Endbereich des Schaftabschnitts 16 eines Arbeitsinstruments 14 gezeigt, der in der Fig. 3 oberhalb einer Eingriffsöffnung 22 dargestellt ist und noch nicht in diese eingeführt und darin noch nicht verrastet ist. Der Endbereich weist einen Durchmesser auf, der kleiner ist als der Durchmesser der Eingriffsöffnung 22. Die Eingriffsöffnung 22 ist in dem Schaftbereich des Rastelements 20 angeordnet und durchspannt dieses vollständig, d.h. ist in Form eines Kanals ausgebildet, der an beiden Seiten offen ist. Durch Einführen des Schaftabschnitts 16 in den Halbtrichter-förmigen Endbereich der Eingriffsöffnung 22 wird das Rastelement 20 entlang seiner Längsachse in Richtung des Sperrelements 34 bzw. in Richtung des Trichteranfangs verschoben (in der Fig. 3 nach links).

Die Eingriffsöffnung 22 und der Endbereich des Schaftabschnitts 16 des Arbeitsinstruments 14 können, wie an anderer Stelle hierin beschrieben, nicht dargestellte, komplementäre Rastnasen und Rastnuten aufweisen und hierdurch eine zusätzliche Rastverbindung bilden. Darüber hinaus wird durch das Federelement 24 eine Rastnase des Rastsystems stets auf den Grund der Rastnut gedrückt, d.h. ein Druck auf den Schaft des Arbeitsinstruments 14 ausgeübt. Hierdurch wird der Hinterschnitt innerhalb des Rastsystems 18 sichergestellt. Das Federelement 24 ist, aus Richtung der Längsachse des Rastelements 20 gesehen, an einem Ende des Rastelements 20 angeordnet. Das Federelement 24 ist als elastischer Formkörper ausgebildet und, im Gegensatz zu dem Rasteelement 20 und dem Sperrelement 34 teilweise außerhalb des Transporteurs 10 angeordnet. Der lateral an der Transporteurseitenwand heraustretende Teil des Federelements 24 kann zur manuellen Betätigung des Rastelements 20 verwendet werden, insbesondere zum Lösen einer zwischen dem Arbeitsinstrument 14 und dem Rastsystem 18 bestehenden Rastverbindung. Wird ein (manueller) Druck auf das Federelement 24 ausgeübt, so wird die Kraft über ein optionales Kraftübertragungselement 64 auf das Rastelement 20 übertragen und das Rastelement 20 entlang seiner Längsachse in dem Inneren des Transporteurs 10 verschoben. Hierdurch wird die klemmende und/oder rastende Verbindung zwischen Eingriffsöffnung 22 und Schaftabschnitt 16 gelöst, sodass das Arbeitsinstrument 14 leicht aus dem Resektoskop 12 entnommen werden kann.

Der Transporteur 10 weist ferner ein Sperrelement 34 auf, das abschnittsweise in einer zweiten Eingriffsöffnung 22 angeordnet ist und abschnittsweise in einem nicht detailliert dargestellten Kanal in dem Verbindungskörper 15. Hierdurch wird das Rastelement 20 auch dann in dem Transporteur 10 gehalten und vor einem Herausfallen geschützt, wenn kein Arbeitsinstrument 14 in der ersten Eingriffsöffnung 22 angeordnet ist. Das Sperrelement 34 ist langgestreckt ausgebildet und quer zur Längsrichtung des Rastelements 20 und parallel zur Längsrichtung des Transporteurs 10 und des Schaftes 46 ausgerichtet. Die für das Sperrelement 34 vorgesehene Eingriffsöffnung 22 ist vorzugsweise als im Wesentlichen paralleler Kanal zu der für das Arbeitsinstrument 14 vorgesehenen Eingriffsöffnung 22 ausgebildet. Ferner ist sie in Längsrichtung des Rastelements 20 breiter als der Durchmesser des Sperrelements 34 um das gewünschte Verschieben des Rastelements 20 in seiner Längsrichtung nicht zu behindern. Vorzugsweise entspricht die zusätzliche Breite, d.h. die Breite die über den Durchmesser des Sperrelements hinausgeht, dem gewünschten Hub des Rastelements 20.

Fig. 5 zeigt eine schematische Schnittdarstellung des Rastsystems 18 eines erfindungsgemäßen Transporteurs 10 aus Längsrichtung des Transporteurs 10, in der die Spülnuten 26 des Sperrelements 34 erkennbar sind. Der erfindungsgemäße Transporteur 10 unterscheidet sich von dem in Fig. 3 und 4 gezeigten mindestens durch die hier gezeigten Spülnuten 26. Andere Merkmale, wie die Anordnung des Sperrelements 34 in einer Eingriffsöffnung 22 oder die Verwendung eines elastischen Formkörpers als Federelement 24 können auch in den Transporteuren 10 der vorliegenden Erfindung Anwendung finden.

Die Spülnuten 26 des Rastelements 20 erstrecken sich parallel zur Längsachse des Rastelements 20 von dem einen Ende des Rastelements 20 zum anderen. Hierdurch wird ein vollständiger Durchfluss von Spülflüssigkeit bei der Reinigung gewährleistet. Die Spülnuten 26 weisen einen U-förmigen Querschnitt auf.

An seinem außerhalb des Verbindungskörpers 15 liegenden Ende weist das Rastelement 20 einen Kopfabschnitt auf, der einen größeren Durchmesser hat, als der sich in das Innere des Transporteurs 10 erstreckende Schaftabschnitt des Rastelements 20. Die dem Schaftabschnitt zugewandte Seite des Kopfabschnitts dient als Auflagefläche für ein Federelement 24, das als metallische Schraubendruckfeder ausgebildet ist. Das Federelement 24 liegt mit seinem anderen Ende auf einem Absatz des Verbindungskörpers 15 auf und übt einen Druck nach außen aus.

Durch das Sperrelement 34 wird das Rastsystem 18 dennoch in dem Verbindungskörper 15 des Transporteurs 10 gehalten. Das Sperrelement 34 ist im Wesentlichen wie das in Fig. 3 und 4 dargestellte Sperrelement 34 ausgebildet. Es greift jedoch nicht in eine komplementäre Eingriffsöffnung ein, sondern in eine Sperrnut 36, die in der Außenwand des Rastelements 20 ausgebildet ist und deren Längsachse quer zur Längsachse des Rastelements 20 ist. Die Sperrnut 36 ist breiter als der Durchmesser des Sperrelements 34, sodass das gewünschte Verschieben des Rastelements 20 nicht behindert wird.

Fig. 6 zeigt eine schematische Schnittdarstellung des Rastsystems 18 eines alternativen, erfindungsgemäßen Transporteurs 10 aus Längsrichtung des Transporteurs 10. Das Rastelement 20 umfasst in hier nicht dargestellter Weise, wie das in Fig. 5 gezeigte, Spülnuten 26, die jeweils geeignet sind, einen Spülkanal 25 zwischen dem Rastelement 20 und dem Verbindungskörper 15 auszubilden.

Im Gegensatz zu dem Transporteur 10 gemäß Fig. 5 ist das Federelement 24 des in Fig. 6 gezeigten Transporteurs 10 auf der einem Entrastungselement 66 entgegengesetzten Seite des Rastelements 20 angeordnet. Der in dem Verbindungskörper 15 für das Rastelement 20 vorgesehene Kanal (Aushöhlung) ist nicht an beiden Seiten des Verbindungskörpers 15 offen, sondern einseitig geschlossen. Das Federelement 24 ist an der geschlossenen Seite dieses Kanals angeordnet und stützt sich einseitig an dem Ende des Kanals ab und mit der anderen Seite an dem Rastelement 20. Zur Abstützung des Federelements 24 weist das Rastelement 20 an diesem Ende Nuten auf, die in Längsrichtung des Rastelements 20 umlaufend um dessen Längsachse ausgebildet sind.

Das Entrastungselement 66 ist ein Betätigungselement (Knopf) mit dessen Hilfe die Rastverbindung zwischen Arbeitselement 14 und dem Rastsystem 18 gelöst werden kann. Dazu ist das Entrastungselement 66 an der dem Federelement 24 gegenüberliegenden Seite des Rastelements 20 angeordnet, d.h. an der Seite des für das Rastelement 20 im Verbindungskörper 15 vorgesehenen Kanals, die nach außen offen ist.

Darüber hinaus unterscheidet sich das in Fig. 6 dargestellte Rastsystem 18 auch dadurch von dem in der Fig. 5 dargestellten, dass der proximale Endbereich des Arbeitselements 14 nicht in einer Eingriffsöffnung 22 des Rastelements 20 sondern in einer Eingriffsnut 23 angeordnet ist. Die Eingriffsnut 23 ist in der Außenwand (Mantelfläche) des Rastelements 20 angeordnet. Die Längsachse der Eingriffsnut 23 verläuft quer zur Längsachse des Rastelements 20.

Das Sperrelement 34 weist einen von außerhalb des Verbindungskörpers 15 zugänglichen Kopfbereich auf, der einen breiteren Durchmesser aufweist, als der innerhalb des Verbindungskörpers 15 und der Sperrnut 36 angeordnete Schaftabschnitt des Sperrelements 34.

Obwohl die vorliegende Erfindung anhand der Ausführungsbeispiele detailliert beschrieben worden ist, ist für den Fachmann selbstverständlich, dass die Erfindung nicht auf diese Ausführungsbeispiele beschränkt ist, sondern dass vielmehr Abwandlungen in der Weise möglich sind, dass einzelne Merkmale weggelassen oder andersartige Kombinationen der vorgestellten Einzelmerkmale verwirklicht werden können, sofern der Schutzbereich der beigefügten Ansprüche nicht verlassen wird. Die vorliegende Offenbarung schließt sämtliche Kombinationen der vorgestellten Einzelmerkmale ein.

**Bezugszeichenliste**

| | | | |
|---|---|---|---|
| 10 | Transporteur | 48 | Schaftrohrsystem |
| 12 | Resektoskop | 50 | Optikführungsrohr |
| 13 | Systemkomponente | 52 | Führungselement |
| 14 | Arbeitsinstrument | 54 | Optik |
| 15 | Verbindungskörper | 56 | Griffteil |
| 16 | Schaftabschnitt | 58 | Griffteil |
| 18 | Rastsystem | 60 | Federbrücke |
| 20 | Rastelement | 62 | Schlitten |
| 22 | Eingriffsöffnung | 64 | Kraftübertragungselement |
| 23 | Eingriffsnut | 66 | Entrastungselement |
| 24 | Federelement | | |
| 25 | Spülkanal | | |
| 26 | Spülnut | | |
| 28 | Ende des Rastelements | | |
| 30 | laterale Transporteurseite | | |
| 32 | kontralaterale Transporteurseite | | |
| 34 | Sperrelement | | |
| 36 | Sperrnut | | |
| 38 | Außenwand | | |
| 40 | Spülkanal | | ***** |
| 42 | Elektrodeninstrument | | |
| 44 | Elektrode | | |
| 46 | Schaft | | |

## Patentansprüche

1. Transporteur (10) für ein Resektoskop (12) für die endoskopische Chirurgie, wobei der Transporteur (10) einen Verbindungskörper (15) und zur Verbindung mit einer Systemkomponente (13) in dem Verbindungskörper (15) ein Rastsystem (18) aufweist, das ein langgestrecktes, vorzugsweise quer zur Längsachse des Transporteurs (10) angeordnetes Rastelement (20) mit einer Eingriffsöffnung (22) oder einer Eingriffsnut (23) und ein Federelement (24) umfasst, **dadurch gekennzeichnet, dass** zwischen Rastelement (20) und Verbindungskörper (15) ein oder mehrere, parallel zur Längsachse des Rastelements (20) angeordnete Spülkanäle (25) ausgebildet sind.

2. Transporteur (10) nach Anspruch 1, **dadurch gekennzeichnet, dass** die Spülkanäle (25) zur Ein- bzw. Ausleitung von Spülflüssigkeit an beiden Enden (28) des Rastelements (20) offen sind.

3. Transporteur (10) nach einem der Ansprüche 1 oder 2, **dadurch gekennzeichnet, dass** durch die Spülkanäle (25) Spülflüssigkeit von einer lateralen Seite (30) des Transporteurs (10) auf die kontralaterale Seite (32) des Transporteurs (10) leitbar ist.

4. Transporteur (10) nach einem der vorangegangenen Ansprüche, **dadurch gekennzeichnet, dass** das Rastelement (20) ein oder mehrere, parallel zur Längsachse des Rastelements (20) angeordnete Spülnuten (26) aufweist.

5. Transporteur (10) nach einem der vorangegangenen Ansprüche, **dadurch gekennzeichnet, dass** der Verbindungskörper (15) ein oder mehrere, parallel zur Längsachse des Rastelements (20) und angrenzend an das Rastelement (20) angeordnete Spülnuten (26) aufweist.

6. Transporteur (10) nach Anspruch 4 oder 5, **dadurch gekennzeichnet, dass** die durch die Spülnuten (26) erzeugten Spülkanäle (40) sich über die gesamte Länge des Rastelements (20) erstrecken.

7. Transporteur (10) nach einem der vorangegangenen Ansprüche, **dadurch gekennzeichnet, dass** die Systemkomponente (13) einen langgestreckten Schaftabschnitt (16) aufweist.

8. Transporteur (10) nach einem der vorangegangenen Ansprüche, **dadurch gekennzeichnet, dass** das Rastsystem (18) zur Verbindung mit dem proximalen Endbereich der Systemkomponente (13) ausgebildet ist.

9. Transporteur (10) nach einem der vorangegangenen Ansprüche, **dadurch gekennzeichnet, dass** der proximale Endbereich der Systemkomponente (13) in die Eingriffsöffnung (22) oder Eingriffsnut (23) des Rastsystems (18) einführbar ist.

10. Transporteur (10) nach einem der vorangegangenen Ansprüche, **dadurch gekennzeichnet, dass** die Systemkomponente (13) ein Arbeitsinstrument (14) ist, vorzugsweise ein Elektrodeninstrument (42) mit einem langgestreckten Schaftabschnitt (16) und einer mit Hochfrequenzstrom beaufschlagbaren Elektrode (44) an seinem distalen Ende.

11. Transporteur (10) nach Anspruch 10, **dadurch gekennzeichnet, dass** der Transporteur (10) zur Steuerung der Längsverschiebung des Arbeitsinstruments (14) ausgebildet ist.

12. Transporteur (10) nach einem der vorangegangenen Ansprüche, **dadurch gekennzeichnet, dass** das Rastsystem (18) ein langgestrecktes Sperrelement (34) umfasst, das quer zur Längsachse des Rastelements (20) in einer Sperrnut (36) in der Außenwand (38) des Rastelements (20) angeordnet ist.

13. Transporteur (10) nach einem der vorangegangenen Ansprüche, **dadurch gekennzeichnet, dass** das Federelement (24) eine Druckfeder ist, vorzugsweise eine Schraubendruckfeder.

14. Resektoskop (12) zur Verwendung in der endoskopischen Chirurgie, **dadurch gekennzeichnet, dass** es einen Transporteur (10) gemäß einem der vorangehenden Ansprüche und vorzugsweise eine in dem Transporteur (10) verrastete Systemkomponente (13) umfasst.

## Claims

1. Transporter (10) for a resectoscope (12) for endoscopic surgery, wherein the transporter (10) has a connection body (15) and, for connection to a system component (13) in the connection body (15), a latch system (18) comprising an elongate latch element (20), which is arranged preferably transversely with respect to the longitudinal axis of the transporter (10) and has an engagement opening (22) or an engagement groove (23), and a spring element (24), **characterized in that** one or more irrigation channels (25), arranged parallel to the longitudinal axis of the latch element (20), are formed between latch element (20) and connection body (15).

2. Transporter (10) according to Claim 1, **characterized in that** the irrigation channels (25) are open at both ends (28) of the latch element (20) in order to allow irrigation liquid to flow in and out.

3. Transporter (10) according to either of Claims 1 and 2, **characterized in that** irrigation liquid is conveyable through the irrigation channels (25) from a lateral face (30) of the transporter (10) to the contralateral face (32) of the transporter (10).

4. Transporter (10) according to one of the preceding claims, **characterized in that** the latch element (20) has one or more irrigation grooves (26) arranged parallel to the longitudinal axis of the latch element (20) .

5. Transporter (10) according to one of the preceding claims, **characterized in that** the connection body (15) has one or more irrigation grooves (26) arranged parallel to the longitudinal axis of the latch element (20) and adjoining the latch element (20).

6. Transporter (10) according to Claim 4 or 5, **characterized in that** the irrigation channels (40) formed by the irrigation grooves (26) extend over the entire length of the latch element (20).

7. Transporter (10) according to one of the preceding claims, **characterized in that** the system component (13) has an elongate shaft portion (16).

8. Transporter (10) according to one of the preceding claims, **characterized in that** the latch system (18) is designed for connection to the proximal end region of the system component (13).

9. Transporter (10) according to one of the preceding claims, **characterized in that** the proximal end region of the system component (13) is insertable into the engagement opening (22) or engagement groove (23) of the latch system (18).

10. Transporter (10) according to one of the preceding claims, **characterized in that** the system component (13) is a work instrument (14), preferably an electrode instrument (42) with an elongate shaft portion (16) and with, at its distal end, an electrode (44) to which high-frequency current can be applied.

11. Transporter (10) according to Claim 10, **characterized in that** the transporter (10) is designed to control the longitudinal displacement of the work instrument (14).

12. Transporter (10) according to one of the preceding claims, **characterized in that** the latch system (18) comprises an elongate blocking element (34) which is arranged, transversely with respect to the longitudinal axis of the latch element (20), in a blocking groove (36) in the outer wall (38) of the latch element (20).

13. Transporter (10) according to one of the preceding claims, **characterized in that** the spring element (24) is a compression spring, preferably a helical compression spring.

14. Resectoscope (12) for use in endoscopic surgery, **characterized in that** it comprises a transporter (10) according to one of the preceding claims and preferably a system component (13) latched in the transporter (10) .

## Revendications

1. Transporteur (10) pour un résectoscope (12) pour la chirurgie endoscopique, le transporteur (10) comprenant un corps de raccordement (15) et un système d'encliquetage (18) pour le raccordement à un composant de système (13) dans le corps de raccordement (15), lequel système d'encliquetage comporte un élément d'encliquetage (20) allongé disposé de préférence transversalement à l'axe longitudinal du transporteur (10) et doté d'une ouverture d'entrée en prise (22) ou d'une rainure d'entrée en prise (23) et un élément ressort (24), **caractérisé en ce qu'**un ou plusieurs canaux de rinçage (25) disposés parallèlement à l'axe longitudinal de l'élément d'encliquetage (20) sont réalisés entre l'élément d'encliquetage (20) et le corps de raccordement (15).

2. Transporteur (10) selon la revendication 1, **caractérisé en ce que** les canaux de rinçage (25) sont ouverts aux deux extrémités (28) de l'élément d'encliquetage (20) pour l'entrée et la sortie de liquide de rinçage.

3. Transporteur (10) selon l'une des revendications 1 ou 2, **caractérisé en ce que** du liquide de rinçage peut être guidé à travers les canaux de rinçage (25) à partir d'un côté latéral (30) du transporteur (10) sur le côté contralatéral (32) du transporteur (10).

4. Transporteur (10) selon l'une des revendications précédentes, **caractérisé en ce que** l'élément d'encliquetage (20) comprend une ou plusieurs rainures de rinçage (26) disposées parallèlement à l'axe longitudinal de l'élément d'encliquetage (20).

5. Transporteur (10) selon l'une des revendications précédentes, **caractérisé en ce que** le corps de raccordement (15) comprend une ou plusieurs rainures de rinçage (26) disposées parallèlement à l'axe longitudinal de l'élément d'encliquetage (20) et de manière adjacente à l'élément d'encliquetage (20).

6. Transporteur (10) selon la revendication 4 ou 5, **caractérisé en ce que** les canaux de rinçage (40) produits par les rainures de rinçage (26) s'étendent sur toute la longueur de l'élément d'encliquetage (20).

7. Transporteur (10) selon l'une des revendications précédentes, **caractérisé en ce que** le composant de système (13) comprend une partie de tige (16) allongée.

8. Transporteur (10) selon l'une des revendications précédentes, **caractérisé en ce que** le système d'encliquetage (18) est réalisé pour le raccordement à la région d'extrémité proximale du composant de système (13) .

9. Transporteur (10) selon l'une des revendications précédentes, **caractérisé en ce que** la région d'extrémité proximale du composant de système (13) peut être insérée dans l'ouverture d'entrée en prise (22) ou la rainure d'entrée en prise (23) du système d'encliquetage (18).

10. Transporteur (10) selon l'une des revendications précédentes, **caractérisé en ce que** le composant de système (13) est un instrument de travail (14), de préférence un instrument à électrode (42) doté d'une partie de tige (16) allongée et d'une électrode (44) pouvant être soumise à un courant de haute fréquence à son extrémité distale.

11. Transporteur (10) selon la revendication 10, **caractérisé en ce que** le transporteur (10) est réalisé pour la commande du déplacement longitudinal de l'instrument de travail (14).

12. Transporteur (10) selon l'une des revendications précédentes, **caractérisé en ce que** le système d'encliquetage (18) comporte un élément de blocage (34) allongé, lequel est disposé transversalement à l'axe longitudinal de l'élément d'encliquetage (20) dans une rainure de blocage (36) dans la paroi extérieure (38) de l'élément d'encliquetage (20).

13. Transporteur (10) selon l'une des revendications précédentes, **caractérisé en ce que** l'élément ressort (24) est un ressort de compression, de préférence un ressort de compression hélicoïdal.

14. Résectoscope (12) destiné à être utilisé dans la chirurgie endoscopique, **caractérisé en ce qu'**il comporte un transporteur (10) selon l'une des revendications précédentes et de préférence un composant de système (13) encliqueté dans le transporteur (10).
